# EUROPEAN PATENT SPECIFICATION

(11) **EP 0 884 329 B1**
(45) Date of publication and mention of the grant of the patent: **25.06.2003**
(21) Application number: 98108374.4
(22) Date of filing: 25.05.1990
(51) Int. Cl.: C07K 16/42, A61K 39/395, C12N 5/10

(54) **Anti-idiotypic antibody which induces an immune response against a glycosphingolipid and use thereof**
Antiidiotypischer Antikörper, der ein Immunantwort gegen ein Glykosphingolipid induziert und seine Verwendung
Anticorps anti-idiotypiques induisant une réaction immunitaire contre un glycosphingolipide, et son utilisation

(30) Priority: 25.05.1989 US 357037
(43) Date of publication of application: 16.12.1998
(62) Divisional of application: 90909554.9
(73) Proprietor: Sloan-Kettering Institute For Cancer Research, New York, New York 10021 (US)
(72) Inventor: Chapman, Paul B., New York, NY 10021 (US); Houghton, Alan N., New York, NY 10021 (US)
(74) Representative: VOSSIUS & PARTNER

(56) References cited:
- EP-A- 0 134 868
- EP-A- 0 293 333
- EP-A- 0 306 995
- WO-A-87/06840
- WO-A-88/08429
- CHAPMAN P.B. ET AL: "An anti-idiotipic monoclonal antibody (mAb) carrying the internal image of GD3 ganglioside" EIGHTIETH ANNUAL MEETING OF THE AMERICAN ASSOCIATION FOR CANCER RESEARCH, 24-27 MAY 1989, PROCEEDINGS SANFRANCISCO, CALIFORNIA, vol. 30, March 1989, page 344 XP002078905
- STARUCH M.J. ET AL: "The adjuvanticity of interleukin 1 in vivo" THE JOURNAL OF IMMUNOLOGY, vol. 130, no. 5, 1983, pages 2191-2194, XP002078906
- SÖDERSTRÖM T. ET AL: "The Escherichia coli K1 capsule shares antigenic determinants with the human ganglioside GM3 and GD3" THE NEW ENGLAND JOURNAL OF MEDICINE, vol. 310, no. 10, 1984, pages 726-727, XP002078907

## Description

R24, an IgG3 mouse monoclonal antibody (mAb) raised against a human melanoma, recognizes the GD₃ ganglioside. The GD₃ ganglioside is abundantly expressed on most melanomas, however, expression of the GD₃ ganglioside on normal tissue is selective and occurs at low concentrations (1). Gangliosides are a subset of glycosphingolipids which in turn are a subset of glycolipids. Glycolipids, as their name implies, are sugar-containing lipids.

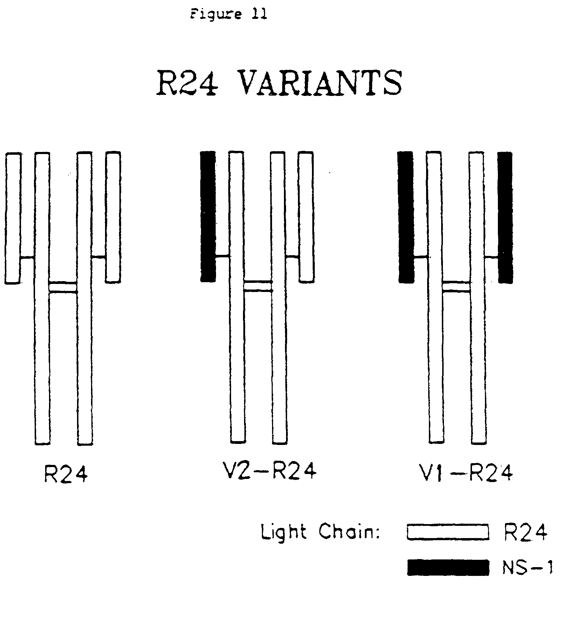

R24 comprises a known mixture of three variant antibody species (Figure 11) designated V1-R24, V2-R24, and R24. The V1-R24 and V2-R24 variants comprise one and two irrelevant antibody light chains, respectively; in contrast, R24 comprises relevant light chains. Generally, light and heavy antibody chains in combination effect specific antibody-antigen binding. However, antibodies comprising irrelevent antibody light chains, unlike antibodies comprising relevant antibody light chains, do not specifically bind an antigen.

In order to avoid confusion, the R24 variant from hereinafter will be referred to as "R24" and R24 comprising the three variant species will be referred to as the "R24 composition." Each variant exhibits differences which imply that the GD₃-binding region (also termed "paratope") of V1-R24, V2-R24, and R24 are altered.

Anti-idiotypic monoclonal antibodies which mimic and possess the internal image of a saccharidic epitope have been developed (2). The saccharidic epitope is a tumor associated globoside, i.e. a glycolipid. However, these anti-idiotypic (monoclonal antibodies do not specifically mimic a glycosphingolipid, a ganglioside, or more particularly the GD₃ ganglioside. Additionally, these anti-idiotypic monoclonal antibodies are not directed against R24 or any antibodies which specifically bind a glycosphingolipid or a ganglioside, let alone the GD₃ ganglioside.

Mice have been primed against capsular antigens of pathogenic bacteria by administration of monoclonal anti-idiotypic antibodies which mimic the polysaccharide capsule of E. coli (a gram negative bacteria) (3). It is generally known that lipopolysaccharides (LPS) is a major component of the outer membrane of gram negative bacteria and is released when bacteria are lysed. LPS consists of a heteropolysaccharide chain linked covalently to a glycolipid. It is important to point out that although these monoclonal anti-idiotypic antibodies were used as a means to prime a subject against a LPS, there is no suggestion of generating monoclonal anti-idiotypic antibodies against R24 or any antibodies which would specifically bind glycosphingolipids or gangliosides, in particular the GD₃ ganglioside.

EP-A-0 306 995 discloses an anti-idiotypic antibody specific to an idiotype which recognizes a human-associated GD3 gangliosidel antigen and is elicited upon recognition of the GD3 antigen by monoclonal antibody MG-21. The anti-idiotypic antibodies of EP-A-0 306 995 are intended to be used to induce an immune response to tumors bearing a specific antigen.

WO-A-88/08429 discloses pharmaceutical compositions and vaccines for the treatment of opportunistic infections in immunodeficient patients. Those compositions and vaccines are based on T-dependent antigens specific for a target infection coupled with a T-cell-independent carrier. T. Söderström et al. (1984), The New England J. of Medicine 310, 726-727; discloses that interleukine 1 enhances in vivo secondary antibody responses in mice to a protein antigen, and that this protein may be an important mediator of host defense responses and of the stimulatory effects of some adjuvants.

Staruch et al. (1983) J. of Immunology 130, 2191-2194 relates to the adjuvanticity of interleukin 1 in vivo. Specifically, interleukin 1 was shown to enhance in vivo secondary antibody responses of mice to a protein antigen. The activity was found to be time and dose dependent. The maximum enhancing effect was obtained with 2000 LAF units of IL1 given two hours after the priming dose of antigen. The observation suggests that this protein may be an important mediator of host defense response, as well as of the stimulatory effects of some adjuvants.

The GD₃ ganglioside is poorly immunogenic (1). Accordingly, the GD₃ ganglioside is an appealing target for cancer immunotherapy because of the commercial utility of designing molecules which would structurally mimic GD₃ and possess dramatically significant immunogenic properties.

The present invention provides the anti-idiotypic antibody designated BEC2 and produced by the hybridoma designated BEC2 hybridoma (ATCC Accession No. HB 10153). The present invention also provides the hybridoma designated BEC2 hybridoma (ATCC Accession No. HB 10153). Additionally, this invention provides a pharmaceutical composition comprising the anti-idiotypic antibody of the invention and a pharmaceutically acceptable carrier. Further, the present invention provides a pharmaceutical composition comprising (a) the anti-GD₃ antibody R24 (ATCC Accession No. HB 8445) labelled with a cytotoxic agent; and (b) the anti-idiotypic antibody of the invention; wherein (a) and (b) are for sequential administration. Finally, this invention provides an immunogenic composition of matter comprising a cytokine and an R24 antibody (ATCC Accession No. HB 8445) attached to a carrier, wherein the cytokine is in an amount effective to stimulate the generation of antibodies in a subject.

The present invention also describes an anti-idiotypic monoclonal antibody which specifically induces an immune response against a glycosphingolipid and more specifically against a ganglioside. Additionally, this invention describes a composition of matter comprising an effective amount of a cytokine and a melanoma ganglioside-specific antibody attached to a carrier. Finally, this invention describes a method of generating the previously-identified anti-idiotypic antibody which comprises: (a) preparing an antibody; (b) purifying the antibody; (c) attaching the antibody onto a carrier so as to produce an immunogen; (d) combining the immunogen with a cytokine so as to produce a novel immunogen-cytokine combination; and (e) injecting the novel immunogen-cytokine combination into a mammal thereby producing the anti-idiotypic antibody.

### Brief Description of the Figures

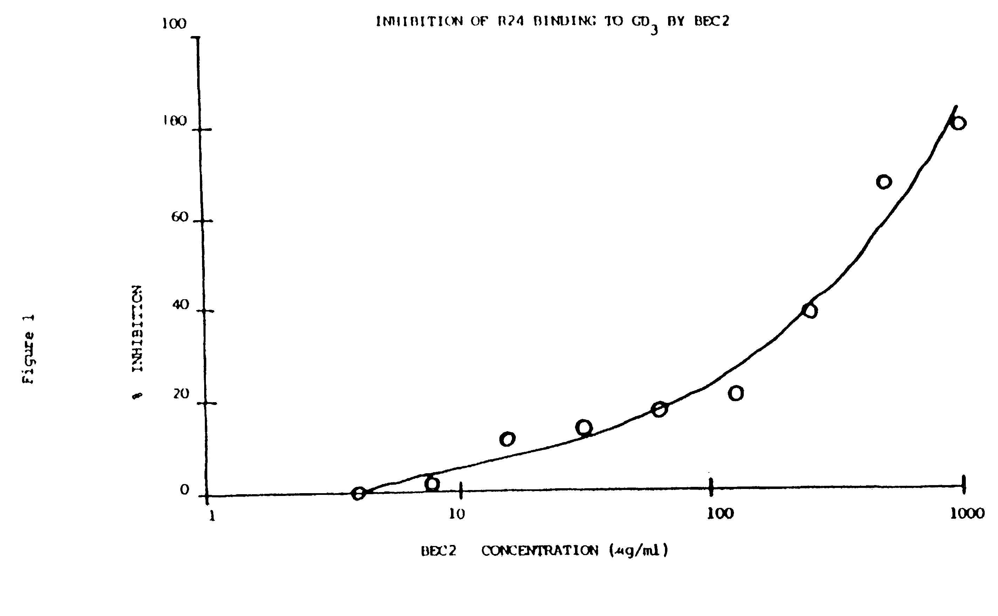

**Figure 1**: Inhibition of R24 binding to GD₃ by BEC2. Dilutions of BEC2 were incubated with 5 µg/ml of biotinylated R24 for 1 hour. The mixture was then plated into wells coated with melanoma gangliosides containing GD₃. Binding of R24 was detected by adding alkaline phosphatase-conjugated avidin for 15 minutes. After washing, substrate (p-nitrophenylphosphate) was added and the absorbance at 410 nm was measured.

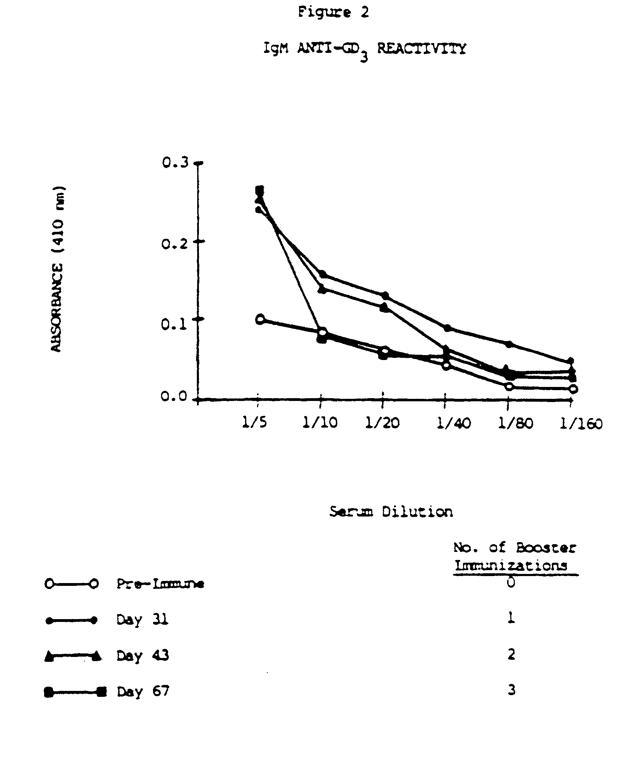

**Figure 2:** Anti-GD₃ reactivity induced in rabbits by immunization with BEC2. Rabbits were inoculated subcutaneously with 100 µl of BEC2 in complete Freund's adjuvant. Subsequent booster immunizations were administered either subcutaneously in incomplete Freund's adjuvant (Days 17 and 31) or intramuscularly without adjuvant (Days 57 and 85). To detect anti-GD₃ rabbit antibodies by ELISA, 96-well plates were coated with purified melanoma GD₃ and blocked with 5% non-fat milk. Dilutions of rabbit serum were added for 1 hour. After washinq, alkaline phosphatase-conjugated anti-rabbit IgG was added. Binding was visualized by adding substrate (p-nitrophenyl-phosphate) and measuring the absorbance at 410 nm.

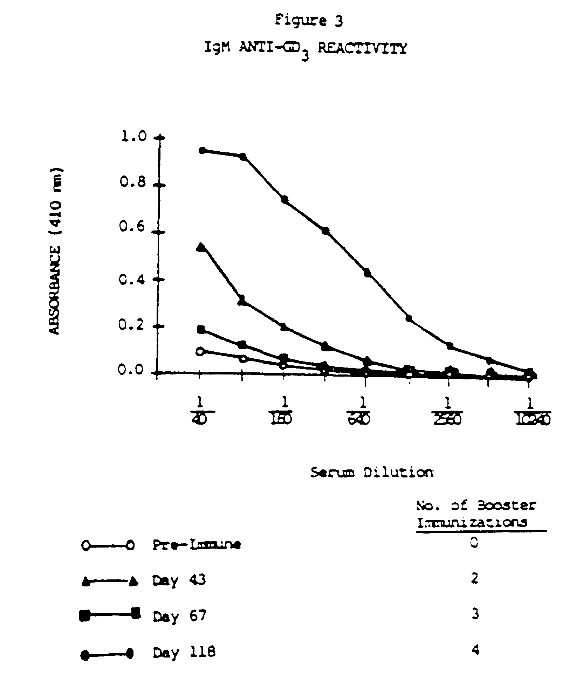

**Figure 3**: Anti-GD₃ reactivity induced in rabbits by immunization with BEC2. Rabbits were inoculated subcutaneously with 100 µl of BEC2 in complete Freund's adjuvant. Subsequent booster immunizations were administered either subcutaneously in incomplete Freund's adjuvant (Days 17 and 31) or intramuscularly without adjuvant (Days 57 and 85). To detect anti-GD₃ rabbit antibodies by ELISA, 96-well plates were coated with purified melanoma GD₃ and blocked with 5% non-fat milk. Dilutions of rabbit serum were added for 1 hour. After washing, alkaline phosphatase-conjugated anti-rabbit IgM was added. Binding was visualized by adding substrate (p-nitrophenyl-phosphate) and measuring the absorbance at 410 nm.

**Figure 4**: Inhibition by BEC2 of R24 binding to GD₃. Data shows significant inhibition of R24 binding to GD₃ by BEC2, this is consistent with BEC2 being an Ab 2b anti-idiotypic mAb.

**Figure 5:** Anti-GD₃ IgG of Rabbit 521 by RIA. Rabbit 521 developed IgG against GD₃ after being immunized with BEC2 mixed with Freund's complete adjuvant. The response was measured by RIA.

**Figure 6:** Anti-GD₃ IgM of Rabbit 543 by ELISA. Rabbit 543 developed IgM after being immunized with BEC2 mixed with Freund's complete adjuvant. The response was measured by ELISA.

**Figure 7:** Anti-GD₃ IgM of Rabbit 545 by ELISA. Rabbit 545 developed IgM after being immunized with BEC2 mixed with Freund's complete adjuvant. The response was measured by ELISA.

**Figure 8:** Anti-GD₃ antibodies induced by BEC2 do not cross-react with other gangliosides. 1µg of various purified gangliosides were applied to strips of nitrocellulose filters and allowed to dry. The strips were blocked in 5% non-fat milk and washed. Strips were incubated in rabbit serum (diluted 1:10) overnight. After washing, peroxidase-conjugated anti-rabbit IgM was added for 2 hours. The strips were again washed and binding was visualized by adding 4-chloro-1-naphthol substrate with H₂O₂.

**Figure 9:** Chromatogram showing that most proteins eluted at a molecular weight of approximately 120kD which is consistent with IgG (145-150 kD) but not consistent with IgM (900 kD).

**Figure 10**: Anti-GD₃ reactivity of NH₄So₄ precipitate from Rabbit 543. Graph shoving the anti-GD₃ reactivity of each fraction. It is clear that only the 120 kD fraction is bound to GD₃.

The present invention describes an anti-idiotypic antibody, such as a polyclonal but preferably a monoclonal antibody, which induces an immune response against a glycosphingolipid. As used herein glycosphingolipids and gangliosides are either naturally-occurring or synthetic molecules.

In one instance the glycosphingolipid is a ganglioside, e.g. GD₃, GD2, GM2, and GM3. GD₃ is preferred.

Generally, an antibody comprises two molecules, each molecule having two different polypeptides, the shorter of which functions as the light chains of the antibody and the longer of which polypeptides function as the heavy chains of the antibody. However, as used herein, antibody is given a functional definition, i.e. any molecule, whether naturally-occurring, artificially induced, or recombinant, which has specific immunoreactive activity. Normally, as used herein, an antibody will include at least one variable region from a heavy or light chain. Additionally, the antibody may comprise combinations of variable regions. The combination may include more than one variable region of a light chain or of a heavy chain. The antibody may also include variable regions from one or more light chains in combination with variable regions of one or more heavy chains.

Accordingly, a fragment of a naturally occurring or recombinant antibody molecule is encompassed within the scope of this invention. As used herein a Fab protein or a F(ab')2 protein which exhibits immunoreactive activity is an antibody. Also, as used herein, an immunologically reactive complex comprising two different polypeptides, the shorter of which function as a light chain and the longer of which function as a heavy chain is an antibody. Further, as used herein, a variable region fragment (Fv region) is also an antibody.

Further, in one example, the immunologically reactive complex may be produced by known genetic engineering methods or otherwise (4, 8, 9, 5, 10).

In another example, the previously described anti-idiotypic monoclonal antibody, which specifically induces an immune response against the GD₃ ganglioside also specifically binds to the binding site of the R24 antibody.

The genetically engineered anti-idiotypic antibody described herein may be derived from any mammal. The genetically engineered anti-idiotypic antibody may also be a chimeric antibody derived from a combination of different mammals. The mammal may be, for example, a rabbit, a mouse, a rat, a goat, or a human. The combination of different mammals includes fragments from human and mouse sources.

The antibody class and sub-class is not critical. Any sub-class may be used to prepare the anti-idiotypic antibodies. The antibodies may also contain fragments from antibodies of different classes and sub-classes, thereby forming a composite. In one aspect , the above-described anti-idiotypic antibody is an IgG class anti-idiotypic antibody.

The previously-described anti-idiotypic monoclonal antibody, is designated BEC2. The present invention also provides a hybridoma which produces the above-described anti-idiotypic antibody. The hybridoma that produces BEC2 is designated BEC2 hybridoma. The BEC2 hybridoma has been deposited pursuant to the Budapest Treaty On the International Recognition Of The Deposit of Microorganisms For The Purposes of Patent Procedure with the Patent Culture Depository of the American Type Culture Collection (ATCC), 12301 Parklawn Drive, Rockville, Maryland 20852 U.S.A. under ATCC Accession No. HB 10153. The present invention further provides an anti-idiotypic antibody produced by the BEC2 hybridoma.

In one embodiment of this invention, the anti-idiotypic antibody is labeled with a detectable marker. The detectable marker may be created by an enzyme reaction, for example, alkaline-phosphatase or horseradish peroxidase.

It would be clear to those skilled in the art that one method of attaching the subject anti-idiotypic antibody to an enzyme, would be by a modification of the periodate method (6). Alternatively, it would be clear to those skilled in the art to attach a ligand to the subject anti-idiotypic antibodies and a receptor of the ligand to the enzyme. A suitable ligand receptor combination is biotin-avidin in accordance with the method of Bayer et al. (7).

Moreover, this invention describes a method of generating the above-described claimed anti-idiotypic antibody, BEC2, which comprises: (a) preparing an antibody, preferably a monoclonal antibody, against a glycosphingolipid, more specifically against a ganglioside, and more specifically against GD₃; (b) purifying the antibody; (c) attaching the antibody, such as R24, to a carrier so as to produce an immunogen; (d) combining the immunogen with a cytokine so as to produce a novel immunogen-cytokine combination; and (e) injecting the novel immunogen-cytokine combination into a mammal thereby producing the anti-idiotypic antibody.

In accordance with the practice of the invention the carrier may be any material which when combined with the antibody is immunogenic. The carrier may be a microbe, a liposome, or a proteosome. In one example the microbe is S. aureus.

Additionally, in accordance with the practice of the invention the cytokine may be an interleukin, preferably interleukin 1. In one example, interleukin 1 is recombinant human interleukin 1.

GD₃ is poorly immunogenic in man. In contrast, the subject anti-idiotypic antibodies are strongly immunogenic.

The present invention provides vaccine comprising the anti-idiotypic antibody BEC2, and a pharmaceutically acceptable carrier. These vaccines are highly potent and commercially useful therapeutics.

Examples of suitable carriers are well known in the art and may include, but are in no way and are not intended to be limited to, any of the standard pharmaceutical carriers such as a phosphate buffered saline solutions, water, emulsions such as oil/water emulsion, and various types of wetting agents. Other carriers may also include sterile solutions, tablets, coated tablets, and capsules.

Typically such carriers contain excipients such as starch, milk, sugar, certain types of clay, gelatin, stearic acid or salts thereof, magnesium or calcium sterate, talc, vegetable fats or oils, gums, glycols, or other known excipients. Such carriers may also include flavor and color additives or other ingredients. Compositions comprising such carriers are formulated by well known conventional methods. buffered saline solutions, water, emulsions such as oil/water emulsion, and various types of wetting agents. Other carriers may also include sterile solutions, tablets, coated tablets, and capsules.

This invention further provides a vaccine for immunizing a human subject against GD₃ gangliosides wherein a suitable dose of the above-described vaccine is to be administered to the subject. Administration of any of the vaccines may be effected by various ways. Suitable means of administration includes intravenous, intraperitoneal, subcutaneous, or intramuscular, topical, or intradermal administration.

Additionally, this invention provides a vaccine for treating a human subject with a neoplastic or preneoplastic condition associated with the expression of glycosphingolipids, for example gangliosides such as GD3 wherein an effective amount of the previously-described anti-idiotypic antibody and a pharmaceutically acceptable carrier is to be administered to the subject. Examples of neoplastic and preneoplastic conditions associated with expression of GD₃ gangliosides include a condition selected from a group consisting of a melanoma, squamous cell carcinoma, glioblastoma, sarcoma, T cell leukemia and lymphoma, Hodgkin's disease, small cell carcinoma of the lung, or brain tumor.

In one example, administration of the previously-described anti-idiotypic antibody is effected by intravenous administration. In another example of the invention, administration of the previously-described anti-idiotypic antibody is effected by intraperitoneal administration. In yet another example, administration of the previously-described anti-idiotypic antibody is effected by subcutaneous administration. In still another example of the invention, administration of the previously-described anti-idiotypic antibody is effected by intramuscular administration. Further, in another example of the invention, administration of the previously-described anti-idiotypic antibody is effected by topical administration. Additionally, in yet another example, administration of the previously-described anti-idiotypic antibody is effected by intradermal administration.

This invention also provides a vaccine for inhibiting a microbial infection, such as an endotoxin-related infection, in a subject which comprises priming the subject with an effective amount of the previously-described vaccine in order to generate an antibody which specifically binds to a glycosphingolipid on a microbial membrane, the antibody and the glycosphingolipid forming a combination and thereby inhibiting the microbial infection.

Additionally, this invention provides a vaccine for inhibiting a malignancy associated with expression of GD₃ gangliosides in a subject such as a human subject wherein the subject is to be primed with an effective amount of the previously-described vaccine in order to generate an antibody which specifically binds to a GD₃ ganglioside, the antibody and the GD₃ ganglioside forming a combination and thereby inhibiting a malignancy associated with expression of GD₃ gangliosides. Examples of malignancies associated with expression of GD₃ gangliosides include a malignancy selected from a group consisting of a melanoma, squamous cell carcinoma, glioblastoma, sarcoma, T cell leukemia and lymphoma, Hodgkin's disease, small cell carcinoma of the lung, or brain tumor.

In one example, the method for measuring expression levels of GD₃ gangliosides in tissue from a subject is effected by determining the amount and distribution of GD₃ in tissue sections from a neoplastic tissue to be tested. The method comprises contacting the tissue to be tested with the R24 antibody and thereby detecting the expression level and distribution of GD₃.

The present invention provides a pharmaceutical composition for treating a subject suffering from a malignancy associated with expression of GD₃ gangliosides on malignant cells wherein a cell killing amount of an anti-GD3 antibody labeled with a cytotoxic agent, such as a radioisotope, a drug, or a heavy metal is to be administered to the subject. The labeled antibody binds to GD3 ganglioside, after which the cytotoxic agent kills the malignant cells to which the GD3 ganglioside is attached. It is important to eliminate unbound antibody labeled with a cytotoxic agent quickly. Elimination may be expedited by contacting the unbound labeled antibody with any of the previously-described anti-idiotypic antibodies. The anti-idiotypic antibodies bind the previously unbound labeled antibody so as to form a complex which may be cleared by excretion from the subject. In this way the subject is treated for the malignancy without undue harm by the cytotoxic agent.

It would be well known to those skilled in the art that "clearing" undesirable composites, in particular the labeled complex, would involve transport of the complex to the spleen, lung, or liver, wherein an Fc region of the antibody within the composite would bind to the Fc receptor of spleen, lung, or liver tissues or cells derived therefrom. After binding to these tissues or the cells derived therefrom, the complex is excreted from the body by physiological or biochemical means.

Examples of a malignancy associated with the expression of GD₃ gangliosides includes any malignancy selected from a group consisting of a melanoma, squamous cell carcinoma, glioblastoma, sarcoma, T cell leukemia and lymphoma, Hodgkin's disease, small cell carcinoma of the lung, or brain tumor.

This invention additionally describes a method of inhibiting the proliferation of cells associated with elevated levels of GD₃ gangliosides in a subject. The method comprises administering to the subject an effective amount of the previously-described vaccine.

Further, this invention provides an immunogenic composition of matter comprising an effective amount of a cytokine, e.g. interleukin 1, preferably recombinant human interleukin 1, and a melanoma ganglioside-specific antibody, such as a R24 antibody, attached to a carrier. Further, the carrier may be a microbe, preferably S. aureus.

Additionally, the present invention describes a method of generating an anti-idiotypic antibody which comprises: (a) attaching an antigen, for example R24, to a microbe thereby obtaining an immunogen; (b) purifying the immunogen; (c) combining the immunogen with interleukin 1 so as to produce an immunogen-interleukin 1 combination; and (d) injecting the immunogen-interleukin 1 combination into mice, preferably syngeneic mice, thereby generating the anti-idiotypic antibody.

Finally, this invention is illustrated in the Experimental Details section which follows. This section is set forth to aid in an understanding of the invention but is not intended to, and should not be construed to, limit in any way the invention as set forth in the claims which follow.

### Experimental Details

### Anti-idiotypic mAbs to R24

We immunized syngeneic (C57B1/6X BALB/C)F1 female mice with purified R24 free of variants V1-R24 and V2-R24. The significance of using syngeneic mice is that the only portion of the R24 molecule which should be recognized as foreign is the paratope. It is standard to mix the immunizing material with an "adjuvant", a potentiator of an immune response, which boosts the immune response of the mouse. The novel adjuvant disclosed herein was made by sticking the R24 onto S. aureus cells and injecting the R24-coated cells along with rh IL-1 (DuPont).

The mice received two weekly boosts. Three days before fusion, mice were injected with 50 µg R24 i.v. Splenocytes were fused with SP2/0 using standard techniques. Hybridomas were screened for the ability to bind to R24 F(ab')₂ fragments using an alkaline phosphatase conjugated goat anti-mouse F_{c}-specific second antibody. Positive colonies were subcloned twice by limiting dilution. Both S. aureus and IL-1 are strong stimulators of B lymphocytes. Using standard hybridoma techniques and fusion of immune mouse spleen cells to the SP2/0 mouse myeloma cell line BEC2 and BEC3 were produced.

### Demonstrating that BEC2 is an Anti-idiotypic mAb

### BEC2 recognizes unique determinants on R24

If BEC2 is indeed an anti-idiotype for R24, it would be expected that BEC2 would bind only to R24 and no other mAb. We have tested the ability of BEC2 to bind to 23 other mAbs using an inhibition assay. No mAb, other than R24, can compete with R24 for BEC2 binding (Table 1). It is notable that V1-R24, the variant with two irrelevant light chains, is not bound by BEC2. This is consistent with the model that BEC2 recognizes the paratope of R24.

The results in Table 1 were obtained using the following method. BEC2 (5µg/ml) was pre-incubated with dilutions of inhibiting mAb for 1 hr. The mixture was then, plated into wells previously coated with R24 F(ab')₂ fragments and blocked with 5% non-fat milk. BEC2 binding was detected by ELISA using an alkaline phosphatase-conjugated second antibody specific for the F_{c} region. BEC2 carries the internal image of the original antigen GD₃

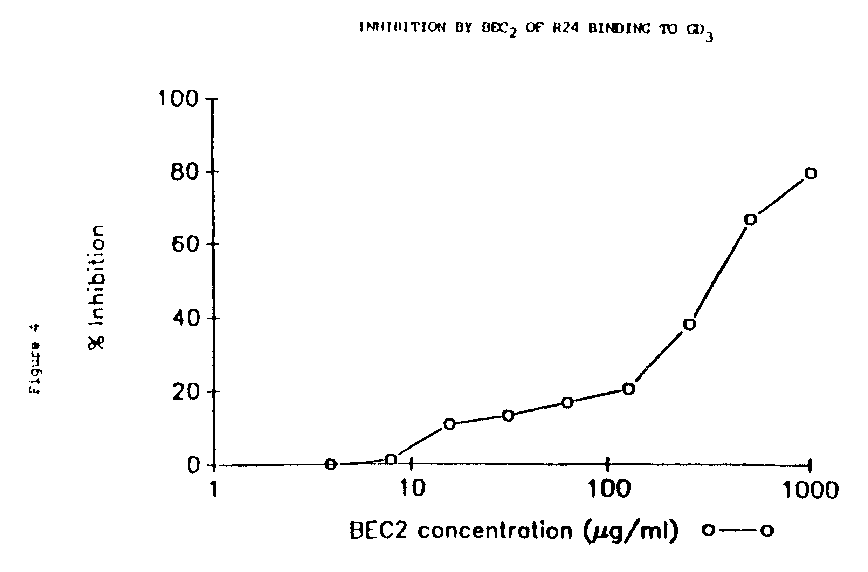

An anti-idiotypic mAb which recognizes the paratope of the immunizing mAb is called an Ab2b anti-idiotypic mAb. One implication of this is that the paratope of BEC2 should mimic GD₃. If one imagines the R24 paratope as a template for GD₃, and both GD₃ and BEC2 fit that template, then BEC2 must resemble GD₃. Using serological assays, we show that BEC2 can inhibit the binding of R24 to GD₃. Figure 4 shows the results on one experiment in which different concentrations of BEC2 were preincubated with a fixed dilution of biotinylated R24. The mixture was added to a well coated with GD₃ and the amount of R24 available for binding was measured by adding alkaline phosphatase-conjugated avidin. The data show significant inhibition of R24 binding to GD₃ by BEC2 which is consistent with BEC2 being an Ab2b anti-idiotypic mAb. In order to directly demonstrate that the paratope of BEC2 mimics GD₃, it is necessary to immunize animale with BEC2 and show that the animals develop antibodies to GD₃.

We immunized four Hew Zealand White Rabbits with BEC2 mixed with Freund's complete adjuvant. The animals received several booster immunizations mixed with Freund's incomplete adjuvant

**Table 1**

| Ability of BEC2 to bind to various monoclonal antibodies as measured by the ability of the monoclonal antibody to prevent binding of BEC2 to R24. Inhibition of BEC2 binding to R24 F(ab')₂ Fragments by a Panel of mAb. | | | | |
|---|---|---|---|---|
| **Antibody** | | **Isotype** | **Specificity** | **IC501** |
| 1. | MOPC104E | M | &1->3 dextrans | 390 |
| 2. | TEPC183 | M | ND2 | 425 |
| 3. | MOPC315 | A | Dinitrophenol ... | >900 |
| 4. | TEPC15 | A | Phosphoryl choline | >900 |
| 5. | MOPC21 | G1 | ND | >700 |
| 6. | HT29-15 | G1 | Adenocarcinoma | >900 |
| 7. | 455 | G1 | EGP receptor | >900 |
| 8. | M111 | G1 | gp110 | 465 |
| 9. | S22 | G1 | ND | >1000 |
| 10. | 2G10 | G1 | gp76 | >1000 |
| 11. | C350 | G1 | gp180 | >500 |
| 12. | UPC10 | G2a | b2-6 fructosan | >900 |
| 13. | TA99 | G2a | gp75 | >900 |
| 14. | A33 | G2a | ND | >900 |
| 15. | M195 | G2a | CD33 | >1000 |
| 16. | F23 | G2a | CD13 | >1000 |
| 17. | MOPC141 | G2b | ND | 485 |
| 18. | OKB7 | G2b | CD21 | >1000 |
| 19. | FLOPC21 | G3 | ND | >900 |
| 20. | Y5606 | G3 | AMP/purine/tri- | |
| | | | ethanolamine | >500 |
| 21. | F36/22 | G3 | Breast Carcinoma | >1000 |
| 22. | 3F8 | G3 | GD2 ganglioside | >1000 |
| 23. | V1-R243 | G3 | GD₃ ganglioside | 425 |
| 24. | R24 | G3 | GD₃ ganglioside | 26 |
| ¹IC₅₀ is the concentration of inhibiting mAb(in lg/ml) required to inhibit 50% of BEC2 binding to R24 F(ab')₂ fragments. ²Not Determined ³A variant of R24 in which both light chains are replaced with MOPC 21 myeloma light chains. This results in a mAb with >40-fold lower affinity for GD₃. | | | | |

over approximately three months. The rabbits were bled periodically and the serum was assayed for ant -GD₃ reactivity using two different assays. The anti-GD₃ IgG response was measured by radioimmunoassay (RIA) using [¹²⁵I]-Protein A. The anti-GD₃ IgM response was measured by ELISA using alkaline phosphatase-conjugated goat anti-rabbit second antibody which was specific for the mu chain. The specificity of both the IgG and IgM response was analyzed by immunoblot assays against several gangliosides. In these assays, [¹²⁵I]-Protein A was used to visualize IgG binding while peroxidase-conjugated goat anti-rabbit (mu chain specific) antiserum was used to visualize IgM binding.

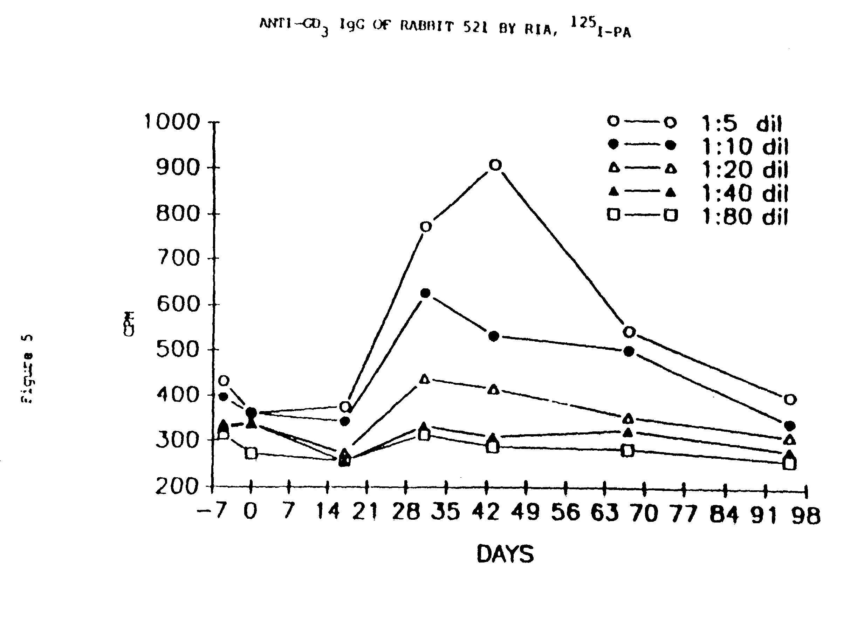

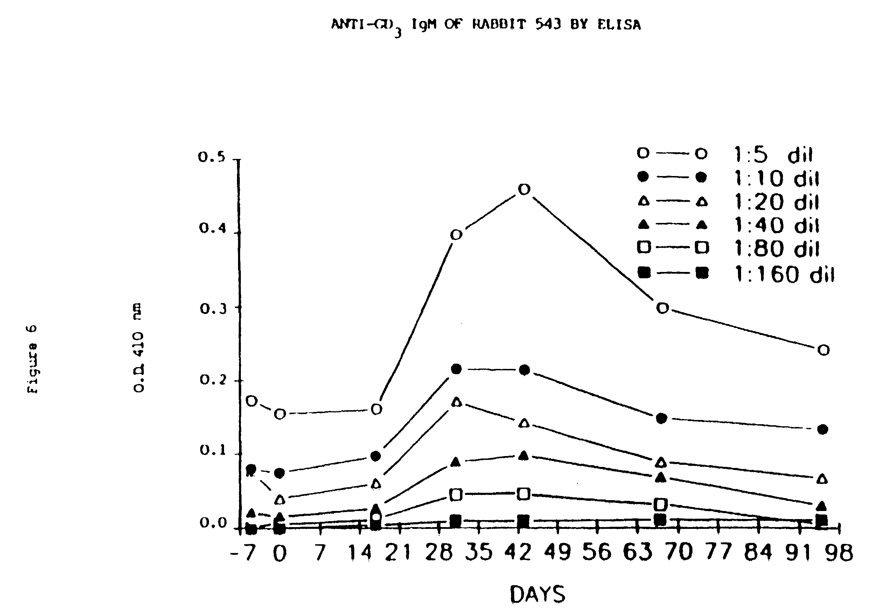

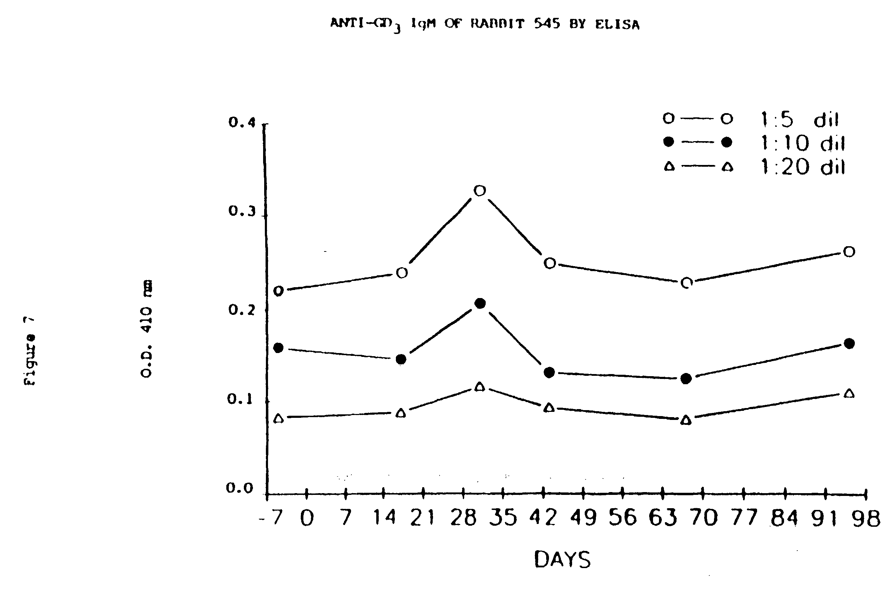

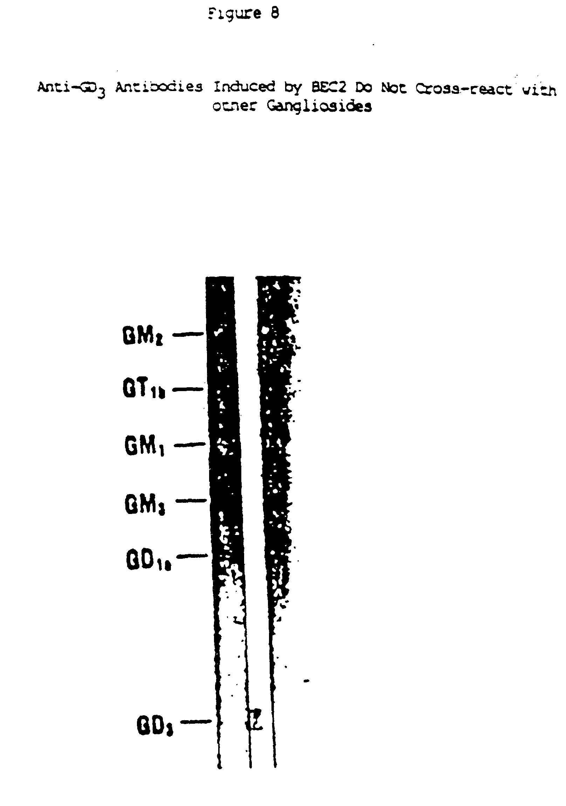

At least three of the four rabbits developed an anti-GD₃ antibody response. By RIA, Rabbit 521 developed IgG against GD₃ (Figure 5) although by immunoblotting, all four rabbits appeared to make anti-GD₃ IgG. By ELISA, rabbits 543 (Figure 6) and 545 (Figure 7) developed IgM. The specificity of this antibody response was analyzed by immunoblot. Figure 8 shows that IgM from rabbit 543 binds to GD₃ but not to the other five gangliosides tested (GM1, GM2, GM3, GD1a, GT1b). As noted above, immunoblotting of serum from all four rabbits showed evidence of IgG binding to GD₃ and this binding was specific.

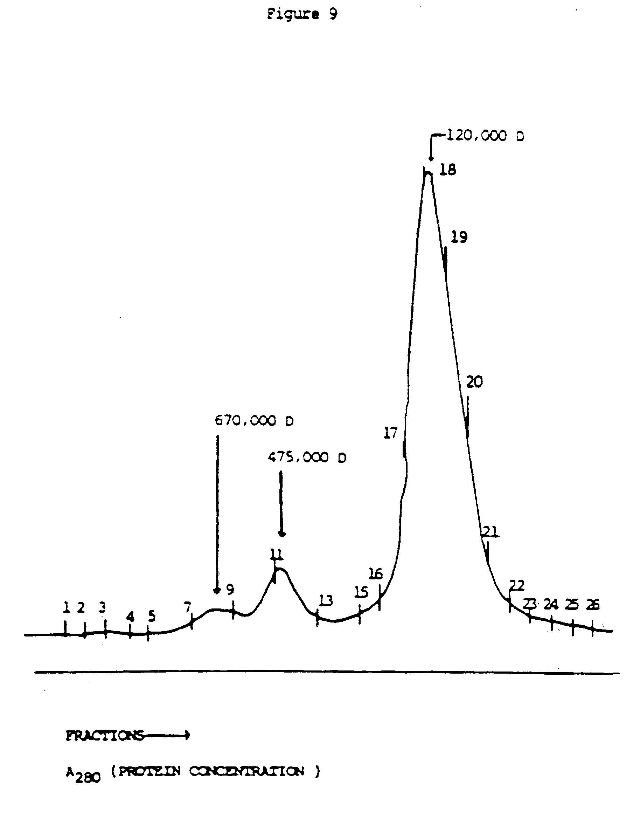

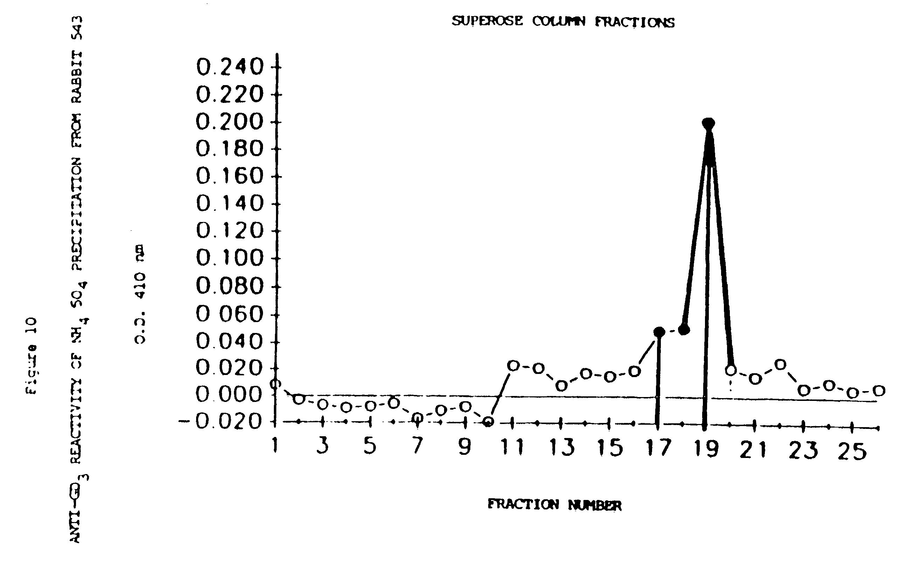

In order to confirm that this anti-GD₃ reactivity was indeed IgG, serum from rabbit 543 was precipitated using 45% NH₄SO₄ and the immunoglobulin salted out was fractionated by size exclusion chromatography (Superose 6 column, Pharmacia). Figure 9 shows that most of the protein eluted at a molecular weight of approximately 120 kD which is consistent with IgG (145-150 kD) but not consistent with IgM (900 kD). Figure 10 shows the anti-GD₃ reactivity of each fraction and it is clear that only the 120 kD fraction bind to GD₃.

### Results

BEC2 binds to the Fab region of R24 and blocks R24 binding to GD₃ gangliosides. Additionally, BEC2 binds to two anti-GD₃ monoclonal antibodies, C5(IgG3) and K9 (IgM). Because BEC2 contains irrelevant light chains BEC2 neither binds to other mouse monoclonal antibodies, including four IgG3 monoclonal antibodies, nor binds to the variant of R24.

BEC2 is an AB2 anti-idiotypic monoclonal antibody for it binds to the portion of R24 which recognizes GD₃. If one imagines the R24 binding site as a template for GD₃, and both GD₃ and BEC2 fit that template, then BEC2 must resemble GD₃. Using serological assays, we have shown that BEC2 does indeed resemble GD₃. BEC2 is a useful way to immunize against GD₃, and thus, important as a vaccine against melanoma and other tumors.

To demonstrate that BEC2 carries an internal image of GD₃, we immunized NZW rabbits with BEC2 and studied pre-immune and immune sera for evidence of anti-GD₃ reactivity. Immunized rabbits developed an antibody response to GD₃ detectable by ELISA and Immunoblot against purified GD₃, and by mixed hemadsorption assay against a GD₃+ human melanoma cell line. Specificity analysis showed that immune sera recognized only GD₃ and not GM₁, GM₂, GM₃, GD₁ₐ, or GT_{1b}, monoclonal antibody BEC2 provides a strategy for active immunization of melanoma patients against GD₃.

Immunization with BEC2 induces high titer, specific IgM and IgG antibodies to GD₃ in rabbits and IgM antibodies in syngeneic (BALB/c x C57B1)F1 mice.

From the experiments, we find that BEC2 is a mouse IgG2b anti-idiotypic mAb directed against R24 and binds the antigen-binding site of R24. Further, immunization of rabbits with BEC2 results in an IgM and IgG response against GD₃. Finally, BEC2 carries the internal image of the ganglioside GD₃, therefore, it is useful for immunization against GD₃ gangliosides.

### REFERENCES

1. T. Tai et al. (1985) Int. S, Cancer, 35: 607.
2. G. Viale et al. (1987) Journal of Immunology, 139:4250.
3. K.E. Stein and T. Soderstrom (1984) Journal of Experimental Medicine, 160:1001.
4. Morrison, S.L. et al. (1988) Clin. Chem. 34:1668.
5. Morrison, S.L. et al. (1987) Ann. N.Y. Acad. Sci. 507:187.
6. Oi, V.T., and Morrison, S.L. (1986) BioTechniques 4:214.
7. Wilson, M.B. and Nakane, P.K. (1978) Immunofluorescence and Related Staining Techniques (Elsevier/North Holland Biomedical Press, Amsterdam) 215.
8. Bayer et al. (1979) Methods in Enzymology, 62:308.
9. Neuberger, et al. (1984) Nature (London), 312:604;
10. Neuberger, M.J. et al. (1985) Nature (London), 314:268-270.

## Claims

1. The anti-idiotypic antibody designated BEC2 and produced by the hybridoma designated BEC2 hybridoma (ATCC Accession No. HB 10153).

2. The anti-idiotypic antibody of claim 1 labelled with a detectable marker.

3. The anti-idiotypic antibody of claim 2, wherein the detectable marker is an enzyme.

4. The anti-idiotypic antibody of claim 2, wherein the detectable marker is a chromophore, a fluorophore, a radioisotope, or a heavy metal.

5. The hybridoma designated BEC2 hybridoma (ATCC Accession No. HB 10153).

6. A pharmaceutical composition comprising the anti-idiotypic antibody of claim 1 and a pharmaceutically acceptable carrier.

7. The pharmaceutical composition of claim 6 which is a vaccine.

8. The vaccine of claim 7 for inhibiting in a human subject a malignancy associated with the expression of GD₃ gangliosides, wherein said vaccine is administered intravenously, intraperitoneally, subcutaneously, intramuscularly, topically or intradermally.

9. The vaccine of claim 8, wherein the malignancy associated with expression of GD₃ gangliosides includes any malignancy selected from the group consisting of a melanoma, a squamous cell carcinoma, a glioblastoma, a sarcoma, T-cell leukemia, T-cell lymphoma, Hodgkin's disease, a small cell carcinoma of the lung, and a brain tumor.

10. The vaccine of any of claims 7 to 9 for inhibiting a microbial infection in a subject, wherein the subject is primed with said vaccine in order to induce an immune resonse against a glycosphingolipid on microbial membrane, thereby inhibiting the microbial infection.

11. A pharmaceutical composition comprising:
(a) the anti-GD3 antibody R24 (ATCC Accession No. HB 8445) labelled with a cytotoxic agent; and
(b) the anti-idiotypic antibody of claim 1;
wherein (a) and (b) are for sequential administration.

12. The pharmaceutical composition of claim 11 for the treatment of malignancies associated with expression of GD₃ gangliosides.

13. The pharmaceutical composition of claim 11 or 12, wherein the cytotoxic agent in (a) is a radioisotope, a drug, or a heavy metal.

14. The pharmaceutical composition of claim 12 or 13, wherein the malignancy associated with expression of GD₃ gangliosides is a malignancy selected from the group consisting of a melanoma, a squamous cell carcinoma, a glioblastoma, a sarcoma, T-cell leukemia, T-cell lymphoma, Hodgkin's disease, a small cell carcinoma of the lung, and a brain tumor.

15. An immunogenic composition of matter comprising a cytokine and an R24 antibody (ATCC Accession No. HB 8445) attached to a carrier, wherein the cytokine is in an amount effective to stimulate the generation of antibodies in a subject.

16. The composition of claim 15, wherein the cytokine is interleukin-1.

17. The composition of claim 15, wherein the carrier is S. aureus.

18. A process for the preparation of the anti-idiotypic antibody of any one of claims 1 to 4, which comprises culturing the hybridoma cell line and isolating the desired antibody, optionally followed by the labeling of the antibody.

19. A process for the preparation of the pharmaceutical composition of claims 6 or 7 or the vaccine of any one of claims 8 to 10 which comprises combining the anti-idiotypic antibody of claim 1 with a pharmaceutical acceptable carrier.

20. A process for the preparation of the pharmaceutical composition of any one of claims 11 to 14 which comprises combining each of the anti-GD₃ antibody labeled with a cytotoxic agent and the anti-idiotypic antibody with a pharmaceutically acceptable carrier.

21. A process for the preparation of the immunogenic composition of any one of claims 15 to 17 which comprises combining the cytokine and the R24 antibody attached to a carrier.

## Patentansprüche

1. Anti-idiotypischer Antikörper, bezeichnet als BEC2 und hergestellt mit dem Hybridom bezeichnet als BEC2-Hybridom (ATCC-Hinterlegungsnr. HB 10153).

2. Anti-idiotypischer Antikörper nach Anspruch 1, der mit einem nachweisbaren Marker markiert ist.

3. Anti-idiotypischer Antikörper nach Anspruch 2, wobei der nachweisbare Marker ein Enzym ist.

4. Anti-idiotypischer Antikörper nach Anspruch 2, wobei der nachweisbare Marker ein Chromophor, ein Fluorophor, ein Radioisotop oder ein Schwermetall ist.

5. Hybridom bezeichnet als BEC2-Hybridom (ATCC-Hinterlegungsnr. HB 10153).

6. Arzneimittel umfassend den anti-idiotypischen Antikörper nach Anspruch 1 und einen pharmazeutisch verträglichen Träger.

7. Arzneimittel nach Anspruch 6, das ein Impfstoff ist.

8. Impfstoff nach Anspruch 7 zur Hemmung einer Malignität in einem menschlichen Individuum, die mit der Expression von GD₃-Gangliosiden assoziiert ist, wobei der Impfstoff intravenös, intraperitoneal, subkutan, intramuskulär, topisch oder intradermal verabreicht wird.

9. Impfstoff nach Anspruch 8, wobei die Malignität, die mit der Expression von GD₃-Gangliosiden assoziiert ist, jegliche Malignität einschließt, die ausgewählt ist aus der Gruppe bestehend aus einem Melanom, Plattenepithelcarcinom, Glioblastom, Sarcom, T-Zell-Leukämie, T-Zell-Lymphom, Hodgkin-Krankheit, kleinzelligem Carcinom der Lunge und Gehirntumor.

10. Impfstoff nach einem der Ansprüche 7 bis 9 zur Hemmung einer mikrobiellen Infektion in einem Individuum, wobei das Individuum mit dem Impfstoff zur Induktion einer Immunantwort gegen ein Glykosphingolipid auf einer mikrobiellen Membran stimuliert wird, wodurch die mikrobielle Infektion gehemmt wird.

11. Arzneimittel umfassend:
(a) den anti-GD₃-Antikörper R24 (ATCC-Hlnterlegungsnr. HB 8445), markiert mit einem cytotoxischen Agens; und
(b) den anti-idiotypischen Antikörper nach Anspruch 1;
wobei (a) und (b) aufeinanderfolgend verabreicht werden.

12. Arzneimittel nach Anspruch 11 zur Behandlung von Malignitäten, die mit der Expression von GD₃-Gangliosiden assoziiert sind.

13. Arzneimittel nach Anspruch 11 oder 12, wobei das cytotoxische Agens in (a) ein Radioisotop, ein Arzneistoff oder ein Schwermetall ist.

14. Arzneimittel nach Anspruch 12 oder 13, wobei die Malignität, die mit der Expression von GD₃-Gangliosiden assoziiert ist, eine Malignität ist, ausgewählt aus der Gruppe bestehend aus einem Melanom, Plattenepithelcarcinom, Glioblastom, Sarcom, T-Zell-Leukämie, T-Zell-Lymphom, Hodgkin-Krankheit, kleinzelligem Carcinom der Lunge und Gehirntumor.

15. Immunogene Zusammensetzung, umfassend ein Cytokin und einen R24-Antikörper (ATCC-Hinterlegungsnr. HB 8445), verknüpft mit einem Träger, wobei das Cytokin in einer Menge enthalten ist, die wirksam ist zur Stimulation der Bildung von Antikörpern in einem Individuum.

16. Zusammensetzung nach Anspruch 15, wobei das Cytokin Interleukin-1 ist.

17. Zusammensetzung nach Anspruch 15, wobei der Träger S. aureus ist.

18. Verfahren zur Herstellung des anti-idiotypischen Antikörpers nach einem der Ansprüche 1 bis 4, umfassend das Züchten der Hybridomzelllinie und Isolieren des gewünschten Antikörpers, gegebenenfalls gefolgt vom Markieren des Antikörpers.

19. Verfahren zur Herstellung des Arzneimittels nach Anspruch 6 oder 7 oder des Impfstoffes nach einem der Ansprüche 8 bis 10, umfassend das Kombinieren des anti-idiotypischen Antikörpers nach Anspruch 1 mit einem pharmazeutisch verträglichen Träger.

20. Verfahren zur Herstellung des Arzneimittels nach einem der Ansprüche 11 bis 14, umfassend das Kombinieren jeweils des anti-GD₃-Antikörpers, markiert mit einem cytotoxischen Agens, und des anti-idiotypischen Antikörpers mit einem pharmazeutisch verträglichen Träger.

21. Verfahren zur Herstellung der immunogenen Zusammensetzung nach einem der Ansprüche 15 bis 17, umfassend das Kombinieren des Cytokins und des mit einem Träger verknüpften R24-Antikörpers.

## Revendications

1. Anticorps anti-idiotypique désigné BEC2 et produit par l'hybridome désigné hybridome BEC2 (n° d'accès ATCC HB 10153).

2. Anticorps anti-idiotypique selon la revendication 1 marqué avec un marqueur détectable.

3. Anticorps anti-idiotypique selon la revendication 2, dans lequel le marqueur détectable est une enzyme.

4. Anticorps anti-idiotypique selon la revendication 2, dans lequel le marqueur détectable est un chromophore, un fluorophore, un radio-isotope, ou un métal lourd.

5. Hybridome désigné hybridome BEC2 (n° d'accès ATCC HB 10153).

6. Composition pharmaceutique comprenant l'anti-corps anti-idiotypique selon la revendication 1 et un entraîneur acceptable sur le plan pharmaceutique.

7. Composition pharmaceutique selon la revendication 6, qui est un vaccin.

8. Vaccin selon la revendication 7 destiné à inhiber chez un sujet humain une malignité associée à l'expression de gangliosides GD₃, où ledit vaccin est administré par voie intraveineuse, par voie intra-péritonéale, par voie sous-cutanée, par voie intramusculaire, par voie topique ou par voie intradermique.

9. Vaccin selon la revendication 8, dans lequel la malignité associée à l'expression de gangliosides GD₃ inclut tout malignité choisie dans le groupe formé par un mélanome, un carcinome malpighien, un glioblastome, un sarcome, une leucémie T, un lymphome T, une maladie de Hodgkin, un cancer pulmonaire à petites cellules, et une tumeur du cerveau.

10. Vaccin selon l'une quelconque des revendications 7 à 9, destiné à inhiber une infection microbienne chez un sujet, où le sujet est sensibilisé avec ledit vaccin afin d'induire une réponse immune contre un glycosphingolipide présent sur une membrane microbienne, inhibant ainsi l'infection microbienne.

11. Composition pharmaceutique comprenant :
(a) l'anticorps anti-GD₃ R24 (n° d'accès ATCC HB 8445) marqué avec un agent cytotoxique ; et
(b) l'anticorps anti-idiotypique selon la revendication 1 ;
dans laquelle (a) et (b) sont destinés à une administration séquentielle.

12. Composition pharmaceutique selon la revendication 11, pour le traitement de malignités associées à l'expression de gangliosides GD₃.

13. Composition pharmaceutique selon la revendication 11 ou 12, dans laquelle l'agent cytotoxique dans (a) est un radio-isotope, un médicament, ou un métal lourd.

14. Composition pharmaceutique selon la revendication 12 ou 13, dans laquelle la malignité associée à l'expression de gangliosides GD₃ est une malignité choisie dans le groupe formé par un mélanome, un carcinome malpighien, un glioblastome, un sarcome, une leucémie T, un lymphome T, une maladie de Hodgkin, un cancer du poumon à petites cellules, et une tumeur du cerveau.

15. Composition immunogène de substance comprenant une cytokine et un anticorps R24 (n° d'accès ATCC HB 8445) fixé à un support, dans laquelle la cytokine est dans une quantité efficace pour stimuler la génération d'anticorps chez un sujet.

16. Composition selon la revendication 15, dans laquelle la cytokine est l'interleukine 1.

17. Composition selon la revendication 15, dans laquelle le support est S. aureus.

18. Procédé pour la préparation d'un anticorps anti-idiotypique selon l'une quelconque des revendications 1 à 4, comprenant la culture de la lignée cellulaire hybridome et l'isolation de l'anticorps choisi, facultativement suivi du marquage de l'anticorps.

19. Procédé pour la préparation de la composition pharmaceutique selon la revendication 6 ou 7 ou du vaccin selon l'une quelqconque des revendications 8 à 10, comprenant la combinaison de l'anticorps anti-idiotypique selon la revendication 1 avec un véhicule acceptable sur le plan pharmaceutique.

20. Procédé pour la préparation de la composition pharmaceutique selon l'une quelconque des revendications 11 à 14 comprenant la combinaison de chacun des anti-GD₃ anticorps marqués avec un agent cytotoxique et l'anticorps anti-idiotypique avec un véhicule acceptable sur le plan pharmaceutique.

21. Procédé pour la préparation de la composition immunogénique selon l'une quelconque des revendications 15 à 17 comprenant la combinaison du cytokine et l'anticorps R24 lié au vehicule.
